# EUROPÄISCHE PATENTSCHRIFT

(11) **EP 0 685 432 B1**
(45) Veröffentlichungstag und Bekanntmachung des Hinweises auf die Patenterteilung: **09.12.1998**
(21) Anmeldenummer: 94114404.0
(22) Anmeldetag: 14.09.1994
(51) Int. Cl.: C02F 3/10

(54) **Schwimmfähiges, verwirbelbares Trägermaterial für biotechnologische Prozesse**
Buoyant fluidisable carrier material for biotechnological processes
Support flottant fluidisable, pour procédés biotechnologiques

(30) Priorität: 03.06.1994 DE 9409077 U
(43) Veröffentlichungstag der Anmeldung: 06.12.1995
(73) Patentinhaber: Ott, Peter, Dr., 01468 Moritzburg (DE); Peukert, Volkmar, Dr., D-01307 Dresden (DE); Koch, Reinhard, D-01609 Gröditz (DE); Augst, Reiner, D-02689 Wehrsdorf (DE)
(72) Erfinder: Ott, Peter, Dr., 01468 Moritzburg (DE); Peukert, Volkmar, Dr., D-01307 Dresden (DE); Koch, Reinhard, D-01609 Gröditz (DE); Augst, Reiner, D-02689 Wehrsdorf (DE)
(74) Vertreter: Hofmann, Klaus

(56) Entgegenhaltungen:
- WO-A-91/11396
- DE-A- 1 459 485
- DE-A- 1 943 848
- DE-A- 2 821 333
- DE-A- 3 928 255
- DE-U- 9 409 077
- US-A- 4 752 429
- DATABASE WPI Week 8711, Derwent Publications Ltd., London, GB; AN 87-075301 & JP-A-62 027 096 (MITSUBISHI PLASTICS) 5. Februar 1987
- DATABASE WPI Week 8713, Derwent Publications Ltd., London, GB; AN 87-089927 & JP-A-62 039 640 (EIWA KASEI KOGYO KK) 20. Februar 1987

## Beschreibung

Die Erfindung betrifft ein schwimmfähiges, verwirbelbares Aufwuchsträgermaterial für Mikroorganismen, welches bei Verfahren und Anlagen zur weitergehenden Wasseraufbereitung, Abwasser- und Schlammbehandlung und der Fermentationstechnik eingesetzt werden kann.

Es ist bekannt, daß in Wasser- und Abwasserbehandlungsanlagen zur Erhöhung der Biomassekonzentration und damit der Reinigungsleistung verschiedenartige Trägermaterialien eingesetzt werden können.

In DD 261 921 A3 ist ein Verfahren zur Herstellung eines körnigen Trägermaterials für biotechnologische Prozesse mit einem spezifischen Gewicht von unter 0,5 g/cm³ beschrieben, welches durch thermische Schrumpfung von Schaumpolystyrenflocken hergestellt wird. In DD 264 887 A1 wird dieses Material an seiner Oberfläche zusätzlich mit Adsorbenzien und/oder inerten Füllstoffen beschichtet.

Beschichtete Trägermaterialien sind beispielsweise auch in DE 29 45 609 A1, DE 30 06 171 B1 und DE 31 05 887 C2 beschrieben.

Allen diesen Materialien haftet der Nachteil an, daß bei deren Herstellung unterschiedlich geformte Teilchen mit verschiedener Größe und Dichte ausgebildet werden, die zu einem unterschiedlichen Auftriebsverhalten führen, wodurch eine sichere und gesteuerte Verfahrensführung unter definierten Bedingungen erschwert wird. Außerdem siedeln sich die Mikroorganismen ausschließlich an der Oberfläche der Trägermaterialien an. Dadurch können sich nur Mikroorganismen mit aeroben oder anaeroben Milieuansprüchen ausbilden. Im belüfteten Wirbelbett werden die Träger nahezu ausschließlich mit aerob lebenden Mikroorganismen besiedelt. Hohlräume, in denen aufgrund von Sauerstoffmangel auch anaerob-anoxische Prozesse ablaufen, z.B. Denitrifikationsprozesse, sind nicht vorhanden.

Bekannt ist auch der Einsatz schaumstoffartiger Trägermaterialien. In DE 31 37 062 werden offenzellige Schaumstoffkörper auf Polyurethanbasis in stückiger Form eingesetzt. In DD 269 610 A1 wird der Einsatz von kompressiblen, offenporigen Trägermaterialien in Belebungsbecken beschrieben. In DE 37 19 418 C1 werden diese Schaumstoffkörper mit einer Klebeschicht versehen und Adsorptionspartikel auf den bzw. in die offenen Poren auf- bzw. eingebracht.
Diese Materialien haben den Vorteil, daß sich nicht nur in den Außen- sondern auch in den Innenschichten Mikroorganismen mit unterschiedlichen Milieuansprüchen ansiedeln, wodurch sich vorteilhafte Reinigungsleistungen dadurch erzielen lassen sollen, daß Nitrifikations- und Denitrifikationsprozesse gleichzeitig ablaufen.
Beim Praxiseinsatz ergeben sich jedoch dadurch Probleme, daß bewachsene Schaumstoffwürfel ein spezifisches Gewicht zwischen 0,9 - 1,1 g/cm³ annehmen. Dadurch ist eine sichere Rückhaltung in den Reaktoren mittels Tauchwänden nicht möglich. Die Rückhaltung kann nur durch Siebe, Netze oder gelochte Platten erfolgen. Dies hat zur Folge, daß der Einsatz dieser Träger nur in Anlagen mit gut funktionierenden Vorreinigung möglich ist und daß der Durchmesser dieser Träger mindestens 2 cm betragen muß. Jede Vergrößerung bedeutet jedoch eine Einschränkung der spezifischen Bewuchsfläche und gleichzeitig eine erhöhte Inanspruchnahme von Reaktorvolumen ohne äquivalente Bioaktivität. Ein weiterer Nachteil der Schaumstoffträger ist es, daß sich die offenen Poren mit wachsendem Bewuchs verschließen und das zu reinigende Medium nicht mehr ausreichend an die inneren Schichten gelangt.

In JP-A-62 027 096 wird ein Körper zur biochemischen Behandlung von Abwasser beschrieben, dem zur Verbesserung der Haftung von Mikroorganismen Nährstoffe, Mikroben, Enzyme, Coenzyme, blähende Mittel zugesetzt werden oder der mit plastischem Material belegt ist.

Aufgabe der Erfindung ist die Schaffung eines Trägermaterials für mikrobiologische Prozesse, welches eine gleichzeitige aerobe sowie anaerob-anoxische Wasserbehandlung ermöglicht. Auch im aeroben Wirbelbett soll ein Teil der Oberfläche durch anaerob-anoxisch lebende Mikroorganismen besiedelt werden. Das Material soll außerdem in einem engen Spektrum definierter Dichte und Größe herstellbar sein und eine große, adsorptiv wirkende Bewuchsfläche besitzen.

Die Aufgabe der Erfindung wird durch ein Material gemäß Anspruch 1 gelöst. Ausgestaltende Merkmale sind in den Ansprüchen 2 bis 6 beschrieben.

Ein polymeres Grundmaterial aus Polyolefinen bzw. deren Co- und Terpolymere, z.B. Polyvinylacetat, wird geschmolzen und unter Zusatz von organischen und/oder anorganischen Stoffen als Treibmittel in einem Extruder mit einer speziell gestalteten Düse zu zylinderförmigen Hohlkörpern mit definierten Abmessungen geformt. Dadurch werden ein geschlossenzelliger Schaumkern und feinporige Zellstrukturen erzeugt. Als Treibmittel werden 0,1 - 2,0 % Bicarbonate mit Zitronensäure und/oder Stärke und/oder Zucker und/oder Aktivkohle eingesetzt. Die innere und/oder äußere Oberfläche kann je nach eingesetztem Treibmittel durch die Blähung porös ausgebildet werden. Die Oberfläche ist strukturiert und profiliert. Die Dichte des Materials soll zwischen 0,4 - 0,98 g/cm³ liegen. Kurz nach Austritt aus der Düse kann die noch nicht erhärtete Oberfläche mit Sorbenzien und/oder Enzymen und/oder Antigenen und/oder anderen biochemischen Präparaten beschichtet werden. Zur Erhöhung der spezifischen Bewuchsfläche ist die verwendete Düse mit Längsrillen versehen, wodurch auf dem Trägermaterial eine geriefte Oberfläche ausgebildet wird. Nach dem Aushärten wird der erzeugte Strang in definierte Längen geschnitten.

Das erzeugte Trägermaterial ist schwimmfähig, gut verwirbelbar sowie mechanisch und biologisch sehr beständig. Es besitzt eine große, adsorptiv wirkende Bewuchsfläche. Das Material läßt sich sehr vorteilhaft bei biotechnologischen Prozessen, insbesondere bei Verfahren zur weitergehenden Abwasserbehandlung mit Stickstoffeliminierung einsetzen. Die Träger können je nach Bedarf in Wirbelbett- oder Festbettreaktoren für aerobe, anaerobe oder anoxische Verfahren genutzt werden. Die Rückhaltung in den Rektoren läßt sich problemlos durch speziell angeordnete Tauchwände realisieren. Ein besonderer Vorteil ergibt sich dadurch, daß je nach Länge der Trägerteilchen in ihrem Inneren Aufwuchsflächen geschaffen werden können, die auch im aeroben Wirbelbett im bewachsenem Zustand nicht ausreichend mit Sauerstoff versorgt werden und sich dort somit anaerob-anoxische Milieubedingungen ausbilden. Dadurch können an einem Trägerteilchen auf engstem Raum aerobe und anaerobe-anoxische Prozesse gleichzeitig ablaufen. Der relative Anteil aerober zu anaerob-anoxischen Bewuchsflächen kann über die Länge der einzelnen Trägerteilchen eingestellt werden. Sind vorwiegend aerobe Prozesse gewünscht, z.B. eine Nitrifikation oder ein aerober BSB-Abbau, dann haben die in diesen Verfahrensstufen eingesetzten Träger eine Länge zwischen 3 - 6 mm. Werden die Träger auf 15 - 25 mm verlängert, dann siedeln sich im Inneren Mikroorganismen an, die zur Denitrifikation befähigt sind.

Das Trägermaterial, seine Herstellung und seine Einsatzmöglichkeiten werden nachfolgend am Beispiel einer Abwasserbehandlungsanlage mit weitergehender Stickstoffeliminierung durch Nitrifikation und Denitrifikation beschrieben.

Polyvinylacetat wird geschmolzen und mit 0,6 % Granulat, welches 40% Bicarbonat mit Zitronensäure als Treibmittel enthält, vermischt. In einem Extruder mit einer speziell gestalteten Düse wird dieses Gemisch zu Hohlzylindern mit in Längsrichtung geriefter Oberfläche zu einem Strang geformt. Dieser hat einen Außendurch-messer von 5 mm und einen Innendurchmesser von 4 mm. Die Riefen sind etwa 0,6 mm tief. Anschließend wird der Strang nach einem Abkühlvorgang in einem Wasserbad in 5 mm lange Stücke geschnitten. Dadurch ergibt sich pro Trägerteilchen eine Bewuchsfläche von über 2,7 cm² und pro m³ Schüttvolumen eine Fläche von über 950 m².

Auf die gleiche Weise wird eine zweite Charge mit einer Trägerlänge von 15 mm hergestellt.

Die zweite Charge wird der Nitrifikationsstufe und die erste Charge der Denitrifikationsstufe einer Abwasserbehandlungsanlage mit Stickstoffeliminierung zu jeweils 45 Vol% zugegeben. Das Trägermaterial der Nitrifikationsstufe wird durch den Eintrag von Druckluft stark verwirbelt während die Trägerteilchen in der Denitrifikationsstufe durch einen langsam laufenden Rührer ständig in Kontakt mit dem zu behandelnden Abwasser gehalten werden. Das aufschwimmende Trägermaterial wird in den jeweiligen Verfahrensstufen durch speziell gestaltete Tauchwände zurückgehalten.

Das Trägermaterial der belüfteten Nitrifikationsstufe wird auf dem gut mit Sauerstoff versorgten Außenflächen vor allem mit aerob lebenden Mikroorganismen, insbesondere durch nitrifizierende Bakterien, besiedelt. Die relativ langsam wachsenden Nitrifikanten bleiben mit dem Träger ständig in dieser Stufe und gewährleisten dadurch in vergleichsweise kurzer Zeit die biochemische Umsetzung von im Abwasser enthaltenen Ammonium in Nitrat. Der mittlere Abschnitt im Inneren der Hohlzylinderteilchen wird aufgrund der geringen Turbulenz stark mit unterschiedlichsten Mikroorganismen bewachsen. Die Sauerstoffversorgung ist in diesem Bereich eingeschränkt. Es siedeln sich hier vorzugsweise solche Bakterien an, die im anaeroben und anoxischen Milieu wachsen können. Die meisten dieser Mikroorganismen sind in der Lage, das an den belüfteten Bewuchsflächen mikrobiell gebildete Nitrat unter Nutzung gelöster Kohlenstoffverbindungen zu molekularem Stickstoff zu denitrifizieren. Somit wird bereits in der Nitrifikationsstufe eine Teilmenge des Stickstoffs eliminiert.
Der vollständige Abbau des Nitrats erfolgt in der nachfolgenden unbelüfteten Denitrifikationsstufe. In dieser sind die Trägerteilchen aufgrund anaerob-anoxischer Milieuverhältnisse vor allem durch Mikroorganismen besiedelt, die in der Lage sind, den an das Nitrat-Ion gebundenen Sauerstoff für Stoffwechselprozesse zu nutzen. Molekularer Stickstoff verläßt die Anlage in gasförmiger Form.

Durch den Einsatz des erfindungsgemäßen Trägermaterials in der Abwasserbehandlungsanlage mit Stickstoffeliminierung konnte die BSB-Raumbelastung im Vergleich zu den bekannten Bemessungsgrößen auf 0,8 - 1,0 kg BSB/m³ d verdoppelt werden, ohne daß nachteilige Auswirkungen auf den Eliminierungsgrad aufgetreten sind. Dieser Effekt wird durch eine spezifisch höhere Biomassekonzentration in den Becken erreicht. So wurden pro m³ Schüttvolumen bis 5 kg Trockensubstanz trägerfixierte Biomasse gemessen. In den Freiräumen zwischen den Trägern konnten nochmals etwa 2,5 kg Trockensubstanz nachgewiesen werden. Dadurch ergab sich eine Gesamtbiomasse von etwa 7 - 8 kg Trockensubstanz. Derartige Konzentrationen lassen sich in konventionellen Abwasserbehandlungsanlagen nicht erreichen. Die zur Abtrennung der Biomasse vom gereinigten Abwasser erforderliche Nachkläreinrichtung konnte aufgrund des hohen Anteils trägerfixierter Biomasse um 35% verkleinert werden. Auch die Menge rückzuführender Biomasse konnte auf Werte um 40% der zufließenden Abwassermenge reduziert werden. Bei niedrigen Zulaufbelastungen wurde auf die Biomasserückführung völlig verzichtet.

## Patentansprüche

1. Schwimmfähiges, verwirbelbares Trägermaterial für biotechnologische Prozesse mit folgenden Merkmalen,
- das Grundmaterial besteht aus polymeren Stoffen,
- das Trägermaterial wird unter Zusatz von organischen und/oder anorganischen Stoffen als Treibmittel in einem Extruder geformt,
- es besitzt einen geschlossenzelligen Schaumkern und feinporige Zellstrukturen,
- die Oberfläche ist strukturiert und profiliert,
- es besitzt die Form von zylinderförmigen Hohlkörpern,
- eine Länge von 3 bis 25 mm,
- einen Außendurchmesser von 3 bis 10 mm,
- einen Innendurchmesser von 2 bis 9 mm und
- eine Dichte zwischen 0,4 - 0,98 g/cm³.

2. Trägermaterial nach Anspruch 1 mit folgenden Merkmalen,
- die Oberfläche ist mit Sorbenzien und/oder Enzymen und/oder Antigenen und/oder anderen biochemischen Präparaten beschichtet.

3. Trägermaterial nach den Ansprüchen 1 und 2 mit folgenden Merkmalen,
- die polymeren Stoffe sind Polyolefine bzw. deren Co- und Terpolymere, z.B. Polyvinylacetat.

4. Trägermaterial nach den Ansprüchen 1 bis 3 mit folgenden Merkmalen,
- Schüttgewicht, Dichte und Auftriebsverhalten sind durch Blähung mittels Zusatz chemischer und/oder organischer Treibmittel in einer Menge von 0,1 - 2,0 % einstellbar.

5. Trägermaterial nach Anspruch 4 mit folgenden Merkmalen,
- die Treibmittel sind Bicarbonate mit Zitronensäure und/oder Stärke und/oder Zucker und/oder Aktivkohle.

6. Trägermaterial nach den Ansprüchen 1 bis 5 mit folgenden Merkmalen,
- die innere und/oder äußere Oberfläche ist porös und/oder sie besitzt Längsrillen.

## Claims

1. A buoyant, fluidisable carrier material for biotechnological processes having the following features:
- the base material consists of polymer substances,
- the carrier material is shaped in an extruder with the addition of organic and/or inorganic substances as the expanding agent,
- it has a closed-cellular foam core and fine-pored cell structures,
- the surface is structured and profiled,
- it is in the shape of cylindrical hollow bodies,
- it is 3 to 25 mm long,
- the outside diameter is 3 to 10 mm,
- the inside diameter is 2 to 9 mm, and
- its density is between 0.4 and 0.98 g/cm³.

2. A carrier material according to claim 1, having the following features:
- the surface is coated with sorbents and/or enzymes and/or antigens and/or other biochemical preparations.

3. A carrier material according to claims 1 and 2, having the following features:
- the polymer substances are polyolefins or there copolymers and terpolymers, e.g., polyvinyl acetate.

4. A carrier material according to claims 1 through 3, having the following features:
- density, bulk density and buoyancy are adjustable by expanding due to the addition of chemical and/or organic expanding agents in an amount of 0.1-2.0%.

5. A carrier material according to claim 4, having the following features:
- the expanding agents are bicarbonates with citric acid and/or starch and/or sugar and/or activated carbon.

6. A carrier material according to claims 1 through 5, having the following features:
- the inside and/or outside surface is porous and/or has longitudinal grooves.

## Revendications

1. Matériau support flottant fluidisable pour procédés biotechnologiques, avec les caractéristiques suivantes:
- le matériau de base se compose de matières polymères,
- le matériau support est formé dans une boudineuse sous adjonction de matières organiques et/ou inorganiques en tant qu'agents moussants,
- il possède une âme en mousse à alvéoles fermées et des structures cellulaires à pores fins,
- la surface est structurée et profilée,
- il présente la forme de corps creux cylindriques,
- une longueur de 3 à 25 mm,
- un diamètre extérieur de 3 à 10 mm,
- un diamètre intérieur de 2 à 9 mm, et
- une densité comprise entre 0,4 et 0,98 g/cm³.

2. Matériau support selon la revendication 1, avec les caractéristiques suivantes:
- la surface est enduite avec des agents de sorption et/ou des enzymes et/ou des antigènes et/ou d'autres préparations biochimiques.

3. Matériau support selon les revendications 1 et 2, avec les caractéristiques suivantes:
- les matières polymères sont des polyoléfines ou leurs co- ou terpolymères, p.ex. de l'acétate de polyvinyle.

4. Matériau support selon les revendications 1 à 3, avec les caractéristiques suivantes:
- la densité apparente, la densité et le comportement sous poussée verticale sont ajustables par gonflement par adjonction d'agents moussants chimiques et/ou organiques en une quantité de 0,1 à 2,0%.

5. Matériau support selon la revendication 4, avec les caractéristiques suivantes:
- les agents moussants sont des bicarbonates avec de l'acide citrique et/ou de l'amidon et/ou du sucre et/ou du charbon actif.

6. Matériau support selon les revendications 1 à 5, avec les caractéristiques suivantes:
- la surface interne et/ou externe est poreuse et/ou est creusée de rainures longitudinales.
